(19) Europäisches Patentamt
European Patent Office
Office européen des brevets

(11) Publication number: **0 132 999 B1**

# (12) EUROPEAN PATENT SPECIFICATION

(45) Date of publication of patent specification: **15.01.92**

(51) Int. Cl.[5]: **C12P 13/22**, //C12N11/02

(21) Application number: **84304966.9**

(22) Date of filing: **20.07.84**

## (54) Process and compositions for preparing phenylalanine.

(30) Priority: **29.07.83 US 518756**

(43) Date of publication of application:
**13.02.85 Bulletin 85/07**

(45) Publication of the grant of the patent:
**15.01.92 Bulletin 92/03**

(84) Designated Contracting States:
**CH DE FR GB IT LI NL SE**

(56) References cited:
**EP-A- 0 048 109**
**EP-A- 0 089 165**
**EP-A- 0 167 058**
**DE-A- 2 841 642**
**DE-A- 3 301 102**
**FR-A- 1 306 640**
**FR-A- 2 027 448**
**FR-A- 2 108 117**
**FR-A- 2 352 827**
**FR-A- 2 509 748**
**GB-A- 1 556 584**
**GB-A- 2 048 266**
**GB-A- 2 084 155**
**US-A- 3 183 170**
**US-A- 3 957 580**

**Biotechnologie and bioengineering, vol. XXV, April 1983, pages 999-1011, John Wiley & Sons, Inc. US, Won-Gi Bang et al "Production of L-tryptophan by Escherichia coli cells"**

(73) Proprietor: **RHONE-POULENC CHIMIE S.A.**
**25, quai Paul Doumer**
**F-92408 Courbevoie(FR)**

(72) Inventor: **Wood, Louis L.**
**11760 Gainsborough Road**
**Rockville Maryland 20954(US)**
Inventor: **Calton, Gary J.**
**5331 Landing Road**
**Elkridge Maryland 2122(US)**

(74) Representative: **Blatchford, William Charles et al**
**WITHERS & ROGERS 4 Dyers Buildings Holborn**
**London, EC1N 2JT(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

Chemical abstracts, vol. 57, 23. July 1962, column 2680a-b, Columbus, Ohio, US. Toshinobu Asai et al "Amino acid fermentation. VII. preparation of L-phenylalanine by bacterial transaminase"

Appl. Environ. Microbiol. vol. 42, n 5 November 1981, S. Yamada et al "Production of L-phenylalamine from trans-cinnamic acid with Rhodotorula glutinis containing L-phenylalanine ammonia lyase activity", pages 773-778

Chemical abstracts, vol. 92, 1980, page 298, abstract n 17989b Columbus, Ohio, US. V.I. Yakovleva et al "Kinetic study of the aspartate-amino-transferase reaction catalyzed by free and immobilized cells of Escherichia coli"

Chemical abstracts, vol. 95, 28 December 1981, page 4, abstract n 220379g, Columbus, Ohio, US. Y Bogaert et al "Reactive polymers containing pendant azetidine or azetidinium functions. 1. syntheisis of poly(acrylic esters)s and poly(methacrylic ester)s of 1-alkyl-3-hydroxy-azetidines and related azetidinum salts"

K. Oishi "The microbial production of amino acids" ed. by K. Yamada et al, Chap. 16 "Phenylalanine and tyrosine", pages 435-452, 1972, John Wiley and Sons, New York, USA

Chemical abstracts, vol. 58, 10 June 1963, column 13094f, Columbus, Ohio, US & JP A 3710672 (Ajinomoto Co. Inc.) 19.8.1962

Chemical abstracts, vol. 74, 18 January 1971, page 37, abstract n 9893d, Columbus, Ohio, US & JP A 7020556 (Tanabe Seiyaku Co Ltd) 13.7.1970

Chemical abstracts, vol 79, 26 November 1973, page 10, abstracts n 126931d & SU A 379581 (Topchiev A.V. Institute of Petrochemical Syntheisis) 20.4.1973

Journal of Membrane Science, vol. 11, n 3, October 1982, pages 365-370, Elsevier Sc. Publ. Co Amsterdam, NL; E. Drioli et al "High temperature immobllized cell ultrafiltration reactors"

## Description

The present invention is primarily concerned with the production of phenylalanine from a precursor thereof, notably phenylpyruvate or phenylpyruvic acid, via transaminase. One embodiment of the invention utilizes immobilized whole cells having transaminase activity to produce phenylalanine from phenylpyruvate. However, according to a further embodiment of the invention, the desired enzyme activity may be obtained by using ruptured or permeabilized cells, as such or as purified fractions thereof, either in the free or immobilized state to obtain phenylalanine.

The production of phenylalanine from phenylpyruvate has been attempted by numerous investigators. There are two possible routes to accomplish this transformation. One is by transamination with an appropriate amine donor while the other is direct reduction amination using a biological energy source such as NAD or NADP.

Sakurai (J. Biochjemistry 43, 851, 1956) attempted the preparation of optically active amino acids via transamination. Sakurai used crude pig heart transaminase (freshly obtained) and found that after 20 hours the yield of phenylalanine reached a maximum of 58% (after subtracting the control value) when aspartic acid was used with a small amount of glutamic acid. When aspartic acid alone was used, the yield was only 50% (after subtracting the control value). Sakurai concluded that both amino acids should be present for maximal yields. He explained this result as a coupled system in which glutamic acid was the amine donor for phenylalanine and the aspartic acid served to regenerate the glutamic acid.

Oishi ("The Microbioal Production of Amino Acids", John Wiley & Sons, K. Yamada et al Eds. 1972, Chap. 16) reviewed the production of phenylalanine from precursor keto acids. He noted a maximum yield of 63.5% phenylalanine was obtained by Asai in screening a large number of microbes which had been dried. This yield was obtained from a strain of Alcaligenes faecalis. The two strains of E. coli surveyed showed a 38.5% and a 53% yield under the reaction conditions used. Asai obtained yields as high as 70.6% when the amine donor was a combination of L-aspartate, L-glutamate and L-leucine. Yields with aspartate in two-fold excess were only 54.5%.

It will be appreciated that the yields noted above with respect to the indicated prior procedures are not suitable for an economic industrial process. Yields in excess of 90% are generally considered essential for a commercially viable process.

Oishi also reported that, by using a coupled enzyme system, Kitai was able to reach 76.8% yield. The coupled system was a yeast alcohol dehydrogenase with beef liver glutamate dehydrogenase and the Serratia marscescene glutamate-phenylalanine amino transferase. The reaction was driven by the removal of acetaldehyde by semicarbazide. Additionally, Kitai was able to drive the reaction to the expected 100% yield of L-phenylalanine by use of a coupled system for reductive amination in which E. coli were used to provide NADP. Glutamate, which served as the amine donor, was the limiting reagent.

Wandrey et al (U.S. Patent 4,304,858) describe a coupled system (with formate dehydrogenase) for the production of phenylalanine form phenylpyruvate while providing exogenous NAD or NADH. The system is also applicable when using alpha-hydroxycarboxylic acid for the precursor as illustrated in U.S. Patent 4,326,031. In both of these systems, however, it is necessary to use the reagent NAD or NADH and to use a coupled system in order to regenerate this expensive and labile material.

The available literature reviewed above indicates that only when using coupled systems are high yields of phenylalanine obtained from phenylpyruvate. When uncoupled systems are used, yields no higher then 71% have been obtained using three different amine donors.

This is expected on the basis of the available literature on transaminase from different sources. For instance, Bulos and Handler (The Journal of Biological Chemistry, Vol. 240, No. 8, pages 3283-3294, August 1965) found that beef heart glutamic-alanine transaminase which catalyzes the reaction:

$$\text{alanine} + \alpha\text{-ketoglutarate} \rightleftharpoons \text{glutamate} + \text{pyruvate}$$

had an equilibrium constant of 2.2. In a system of 0.1 m alanine and 0.15 m α-ketoglutarate, the formation of glutamate would be limited to 70%. Henson and Cleland (Biochemistry 3, pages 338-345, 1964) determined that pig heart glutamic oxaloacetic transaminase which catalyzes the reaction:

α-ketoglutarate + L-aspartate

⇄

oxaloacetate + glutamate

had an equilibrium constant of 0.16-0.17. Thus, a system of 0.1 m a-ketoglutarate and 0.15 m L-aspartate would reach an equilibrium at 32% conversion of the α-ketoglutarate to glutamate.

Canellakis and Cohen (J. Biol. Chem. 222, 53-62, 1956) examined dog liver tyrosine-α-ketoglutaric acid transaminase which catalyses the reaction:

p-hydroxyphenylpyruvic acid + L-glutamic acid

⇄

L-tyrosine + α-ketoglutaric acid

and found that equilibrium conditions were not attained after 3 hours and that the equilibrium is in favor of the formation of glutamic acid and p-hydroxyphenylpyruvic acid.

An important object of the invention is to provide a process for the production of phenylalanine in high yields from phenylpyruvic acid or phenylpyruvate via transaminase. A more specific object is to prepare phenylalanine by such a process which involves a single step, with a single amine donor and requiring neither a coupled system nor the addition of expensive cofactor reagents such as NADP or NAD. Other objects will also be hereinafter apparent.

According to the invention, a process for preparing phenylalanine comprises contacting phenylpyruvic acid or phenylpyruvate with immobilized E.coli cells having aspartate transaminase activity in the presence of an amine donor comprising aspartic acid or a combination of aspartic acid and glutamic acid. The immobilized cells may be whole cells or cells which have been ruptured or permeabilized so as to release their transaminase activity.

In our above-mentioned earlier applications, we have described the preparation and use of compositions comprising whole cells having enzymatic activity wherein the cells are immobilized by means of an insoluble, cross-linked polymer obtained by curing a polyazetidine prepolymer, carboxymethyl cellulose, polyurethane hydrogel prepolymer or polymethylene isocyanate. Preferably the immobilizing polymer is a polyazetidine polymer although the other disclosed polymers may be used. Advantageously the immobilized cells are coated onto beads or other particulate material.

The present invention contemplates the use of immobilized cell compositions as described in said earlier applications, as well as other forms of immobilized cells, provided the immobilized cells have transaminase activity. According to the invention, phenylalanine is produced by contacting a phenylalanine precursor, specifically phenylpyruvate or phenylpyruvic acid, with the immobilized cell composition having transaminase activity in the presence of an amine donor, so that the precursor is converted to phenylalanine. The literature indicates that small amounts of pyridoxal-5-phosphate (P5P) are required by the transaminase as a co-factor. This material (P5P) is also used in the present process in conventional co-factor amounts.

The precursor may be used in the form of the free acid or as a salt thereof, e.g. the ammonium or alkali metal salt. Preferably the donor is used in excess and it appears that higher yields are obtained as the excess is increased up to, for example, 30-50% excess or even more.

The reaction conditions used for carrying out the transaminase reaction according to the invention can be widely varied, as will be understood by those in the art. For example, an aqueous solution of the precursor can be passed through a column containing the immobilized cells containing transaminase activity and the amine donor. Optimum ratios of precursor to donor and to cells, and other operating conditions, can be readily determined for any specific situation without undue experimentation. Typically, however, the ratio of the amine donor to the precursor will be at least 1:1 and preferably 1.1:1 or higher,

e.g., 3:1. A preferred ratio is 1.5-2 parts donor per part precursor, parts being on an equivalent weight basis.

Acid or alkaline pHs may be used although there will generally be a readily determined optimum pH for any particular set of conditions. Usually it is desirable to use a pH above 4, and generally one in the range of 5-10, although pHs outside these ranges may also be used. Temperatures of 30° to 40°C normally will be used although any temperature below transaminase deactivation can be used.

The invention is illustrated by the following examples:

Example 1

Saccharomyces carevisiae, E. coli, Alcaligenes faecalis and Pseudomonas dacunhae cells were immobilized in separate batches with polyazetidine as described in Serial No. 465,551 (see, for instance, Example 8 thereof) by mixing equal parts of cell paste and aqueous polyazetidine solution (Hercules Polycup 172), stirring to homogenity at 25°C by hand mixing with a wooden stick. This mixture was dispersed on Amberlite ion exchange beads which had been air-dried. The thin film of paste/prepolymer mixture on the beads was allowed to air dry at 25°C. One ml of each group of beads containing 0.2 grams of microbial cells per ml of beads was then placed into a 50 ml Erlenmeyer flask containing 25 ml of a 0.1 M aqueous solution of sodium pyruvate and either L-glutamic acid, L-aspartic acid or mixture thereof as amine donor and 0.1 mM of P5P. These were then compared under otherwise similar conditions. The results in terms of phenylalanine (PHE) produced were determined by HPLC analysis of the supernatant after 17 hours of shaking and are presented below in Table I.

Table I

Transamination of Phenylpyruvic Acid (PPA) to Yield Phenylalanine (PHE)

| Microbe | Amine Donor: | Concentration PHE Formed ASP & GLU | GLU | ASP |
|---|---|---|---|---|
| Saccharomyces cerevisiae | | --- | .012 M | <.002 M |
| E. coli | | .024 M | .027 M | .013 M |
| A. faecalis | | .01 M | .014 M | <.002 M |
| P. dacunhae | | .024 M | .028 M | <.002 M |

(The references to "ASP" and "GLU" above represent L-aspartic acid and L-glutamic acid, respectively.)

The foregoing example demonstrates that whole cells immobilized as described and having transaminase activity may be effectively used to produce phenylalanine from phenylpyruvate precursor when an appropriate amine donor is employed.

In the control using P. dacunhae and A faecalis in the fresh, wet free state (unruptured and unpermeabilized), negligible transaminase activity was noted. However, on rupturing the cells or by permeabilizing them, activity was substantially increased. This is surprising because the literature indicates that dried cells, which would normally be considered lysed or permeabilized, do not give commercially acceptable conversions.

Accordingly, the use of ruptured or permeabilized cells to prepare phenylalanine constitutes a further aspect of the invention. Various techniques may be used to rupture or permeabilize the cells for use

according to the invention. For example, the cells may be ruptured by sonication or grinding as known in the art. Alternatively the cells may be permeabilized by chemical treatment, e.g. by treatment with a permeabilizing surfactant such as Triton X100. These treatments apparently allow the phenylpyruvate or phenylpyruvic acid to more readily contact the enzyme and thus improve activity when the microorganism is immobilized.

The use of ruptured cells, and the effect of pH and amine donor level in the results are described in the following example:

Example 2

This Example shows the effect of pH on transaminase activity using E.coli whole cells immobilized in bead form as in Example 1. Three experiments were conducted using 2 ml of beads incubated in 15 ml of .1 M PPA, 0.1 mM P5P and .15 M ASP at 37° C for 24 hours. The pH was adjusted with 1 N NaOH or 1 N HCl.

Table III

| Experiment 1 Immobilized Cells | | Experiment 2 Immobilized Cells | | Experiment 3 Free Cells | |
|---|---|---|---|---|---|
| pH | 24 hours % Conv. | pH | 4 hours Activity (units)* | pH | 4 hours Activity (units) |
| 5.0 | 95.9 | 3 | 32 | 3 | 78 |
| 5.5 | 96.5 | 4 | 99 | 4 | 94 |
| 6.0 | 95.0 | 5 | 219 | 5 | 123 |
| 6.5 | 95.9 | 6 | 212 | 6 | 601 |
| 7.0 | 95.1 | 7 | 217 | 7 | 598 |
| 8.0 | 94.7 | 8 | 207 | 8 | 571 |
| | | 9 | 209 | 8.4 | 586 |
| | | 10 | 156 | 9 | 637 |
| | | | | 10 | 115 |

*A unit is 1 micromol per hour per gram of wet cells.

The data in Table III shows that high yields of PHE similar to those obtainable with ruptured free cells can be obtained using immobilized E. coli.

Example 3

Table IV below provides the results in terms of yield of PHE obtained using immobilized E. coli in a continuous column operation (300 ml, 3.5 x 70 cm) with 0.1 M PPA, 0.15 M ASP and 0.1 mM P5P.

Table IV

| | Experiment 1 | Experiment 2 |
|---|---|---|
| Day 1 | 95.1 | 100 |
| 8 | 91.2 | |
| 9 | 87.9* | |
| 10 | 92.1 | |
| 11 | 92.1 | 92 |
| 12 | 94.2 | |
| 13 | 95.1 | |
| 14 | 96.1 | |
| 15 | 94.8 | |
| 16 | 96.8 | |
| 17 | 95.8 | 85* |
| 18 | 95.3 | |
| 35 | | 91 |
| 42 | | 93 |
| 43 | | 97 |
| 45 | | 100 |

*It should be noted that occasional fluctuations in flow rates may show a reduced activity or yield on such occasions. However, the important factor is the maximum yield which is shown as this is indicative of the full potential of the process exemplified.

Example 4

While polyazetidine polymer is preferred for immobilizing the microorganisms for use herein, the invention contemplates the possibility of using any other suitable immobilizing substrate. As representative of such alternatives, there may be mentioned such materials as polyacrylamide, Kappa-carrageenan, hollow fiber devices, Hypol or XAD coated beads. These materials have been shown to give excellent yields although the activity of the immobilized cells may vary from one immobilizing substrate to another. The results obtained in terms of yields and activities, using different systems involving immobilized E. coli, are shown below in Table V. The process used involved continuous flow onto a column of immobilized cells as described of an aqueous solution of 0.1 M PPA, 0.15 M ASP and 0.1 mM P5P at a pH 8.3-8.5 (adjusted with $NH_4OH$) at 37°C. Flow varied according to column activity and space occupied. Equilibrium was reached at optimal flow prior to taking readings.

Table V

| Cell Immobilization Method | Max. Yield Observed | Activity (Units)* |
|---|---|---|
| E. coli coated on XAD beads with Polycup | 91 | 34 |
| E. coli coated on IRA938 beads with Polycup | 98 | 63 |
| E. coli with HYPOL foam | 95 | 53 |
| E. coli with Kappa-carrageenan gum | 100 | 29 |
| E. coli in a hollow fiber device | 91 | 82 |

---

*1 unit of activity is defined as 1 μ mole/hr/g cells (wet wt.) at maximum conversion.

Of the materials referred to in Table V, XAD is a macroreticular styrene-divinylbenzene resin; IRA 938 is an ion exchange bead resin comprising styrene-divinylbenzene containing tertiary amine substituents; and Hypol is a polyurethane foam. The Kappa-carrageenan gum was cut into particles before use. The hollow fiber device was a commercially available item.

According to still another feature of the invention, it has been found that the yield of phenylalanine (PHE) from phenylpyruvic acid (PPA) as such or as a salt thereof, e.g. sodium phenylpyruvate, can be greatly increased by contacting the starting material with an aromatic transaminase, preferably an aspartate transaminase, which provides an equilibrium constant (Keg) for the reaction which is greater than 5, preferably greater thin 10. The enzyme may be used as such or in the form of immobilized or free cells containing the same.

Review of the literature on aromatic transamination shows that three major transaminating enzymes exist in E. coli. These are transaminase A which is actually two enzymes one of which is aspartate transaminase and the other of which is a tyrosine repressible aromatic transaminase, and transaminase B. Both of the transaminase A enzymes catalyze the formation of tryptophan, phenylalanine and tyrosine. Transaminase B catalyzes the formation of isoleucine, valine, norleucine and norvaline.

The aspartate transaminase, i.e. one of the two enzymes found in transaminase A, has been mapped on the E. coli chromosome at 20 min. and designated as an aspC mutation. The tyrosine repressible amino transferase is at 80 min. on the E. coli map and is designated tyrB.

As part of the present invention, it has been determined that the aspartate transaminase in certain E. coli (such as E. coli ATCC 11303) is enhanced and that the equilibrium constant of this enzyme is >10 thus allowing yields of phenylalanine greater than 95% when used in the reaction:
phenylpyruvate + aspartate = phenylalanine + oxaloacetate

In assays with E. coli mutants from the Yale collection the difference in the Keg for the different transaminases can be seen. Thus, while at 54 hours the ilvE mutant showed 20% and the tryB mutant showed 33% conversion of phenylpyruvate acid to phenylalanine, the aspC showed 62% and E. coli 11303 showed 66% conversion in a free cell assay with glutamate as the amine donor.

When the free cell assay was carried out using 70% aspartic acid and 30% glutamic acid the conversion of phenylpyruvic to phenylalanine at 54 hours was 20% for the ilvE mutant, 57% for the tryB

mutant, 89% for E. coli 11303 and 91% for the aspC mutant.

The results for 70% aspartic acid/30% glutamic acid as the amine donor were nearly the same as those obtained when only L-aspartic acid was the amine donor. The ilvE mutant gave 19% conversion at 54 hours, the tyrB gave 57%, the aspC 100% and E. coli 11303 102%. It can thus be seen that aspartate transaminase is capable of giving much higher yields than expected on the basis of the literature and that these yields are significantly greater than those previously reported. Thus, as a further feature of the invention, it is proposed that phenylalanine be prepared from phenylpyruvic acid, or its equivalent, by contacting the same with aspartate transaminase having an equilibrium content of >5, preferably >10. Advantageously E. coli 11303 is used for this purpose but other types of cells, free or immobilized, may also be employed.

In some cases, it may also be convenient to include a carboxylase in the reaction to meet the desired equilibrium constant of, for example, >10. The use of E. coli 11303 with its inherently enhanced aspartate transaminase activity is itself adequate to satisfy the indicated limitation as to the equilibrium constant. On the other hand there may be situations where the transaminase is not by itself able to meet the required Keg limit. In that case, carboxylase may also be used to drive the reaction to an equilibrium value of >10.

From an examination of the Keg for the reaction involved, determined by the equation:

$$Keg = \frac{[PHE][OX]}{[Asp][PPA]}$$

where OX is oxaloacetate and PHE, Asp and PPA are as aforesaid, it can be seen that the reaction may be driven towards completion by removal of one of the reactants. Oxaloacetate dicarboxylase catalyzes the reaction:

oxaloacetate → pyruvate + $CO_2$.

This reaction coupled with the transamination can effectively remove oxaloacetate from the equilibrium.

Analysis of the effluent stream from immobilized cells in a column such as the one shown in Example 4 shows that it typically contain 35 mM oxaloacetate, 65 mM pyruvate, 5 mM aspartate, 95 mM phenylalanine and 5 mM phenylpyruvate. The equilibrium constant calculated from this data is 13.3 for the production of phenylalanine from aspartic acid and phenylpyruvate.

It will be appreciated that various modifications may be made in the invention described herein.

## Claims

**1.** A process for preparing phenylalanine which comprises contacting phenylpyruvic acid or phenylpyruvate with immobilized E.coli cells having aspartate transaminase activity in the presence of an amine donor comprising aspartic acid or a combination of aspartic acid and glutamic acid.

**2.** A process according to claim 1, wherein the cells are immobilized by polyazetidine polymer.

**3.** A process according to claim 2, wherein the immobilized cells are coated onto beads or other particulate materials.

**4.** A process according to any preceding claim, wherein excess amine donor is employed and the pH is above 4.

**5.** The process of claim 4, wherein the excess of amine donor is at least 50% and the pH is 5-10.

**6.** A process for preparing phenylalanine which comprises contacting phenylpyruvic acid or phenylpyruvate with immobilized E.coli cells having aspartate transaminase activity in the presence of an amine donor, comprising aspartic acid or a combination of aspartic acid and glutamic acid, said cells having been ruptured or permeabilized in order to increase contact between the enzyme and the phenylpyruvic acid or phenylpyruvate.

**7.** A process according to claim 6, wherein the cells have been ruptured by sonicating.

**8.** A process according to claim 6, wherein the cells are permeabilized by treatment with a surfactant.

9. A process according to any preceding claim including adding pyridoxal-5-phosphate.

10. A process according to any preceding claim, wherein the cells are E.coli cells having enhanced aspartate transaminase activity, and an equilibrium constant > 10.

11. A process according to claim 10, wherein the cells are E.coli ATCC 11303 cells.

12. A composition comprising immobilized E.coli cells having aspartate transaminase activity, an amine donor comprising aspartic acid or a combination of aspartic acid and glutamic acids and phenylpyruvic acid or phenylpyruvate.

13. A composition according to claim 12, wherein the cells are immobilized with polyazetidine.

14. A composition suitable for use in converting phenylpyruvic acid or phenylpyruvate to phenylalanine comprising immobilized E.coli cells having aspartate transaminase activity, said cells being ruptured or permeabilized by chemical treatment.

**Revendications**

1. Procédé pour préparer de la phénylalanine, qui comprend la mise en contact de l'acide phénylpyruvique ou d'un phénylpyruvate avec des cellules de E. coli immobilisées et douées d'une activité d'aspartate transaminase, en présence d'un donneur d'amine comprenant l'acide aspartique ou une combinaison de l'acide aspartique et de l'acide glutamique.

2. Procédé selon la revendication 1, dans lequel les cellules sont immobilisées à l'aide d'un polymère de type polyazétidine.

3. Procédé selon la revendication 2, dans lequel les cellules immobilisées sont appliquées en revêtement sur des perles ou sur d'autres matières particulaires.

4. Procédé selon l'une quelconque des revendications précédentes, dans lequel on utilise un excès de donneur d'amine et le pH est supérieur à 4.

5. Procédé selon la revendication 4, dans lequel l'excès du donneur d'amine représente au moins 50 %, et le pH se situe entre 5 et 10.

6. Procédé pour préparer de la phénylalanine, qui comprend la mise en contact de l'acide phénylpyruvique ou d'un phénylpyruvate avec des cellules immobilisées de E. coli douées d'une activité d'asparte transaminase, en présence d'un donneur d'amine comprenant l'acide aspartique ou une combinaison de l'acide aspartique et de l'acide glutamique, lesdites cellules ayant été soumises à rupture ou à perméabilisation d'augmenter le contact entre l'enzyme et l'acide phénylpyruvique ou le phénylpyruvate.

7. Procédé selon la revendication 6, dans lequel les cellules ont été rompues par traitement aux (ultra)-sons.

8. Procédé selon la revendication 6, dans lequel les cellules sont perméabilisées par traitement à l'aide d'un surfactant.

9. Procédé selon l'une quelconque des revendications précédentes, comprenant l'addition de pyridoxal-5-phosphate.

10. Procédé selon l'une quelconque des revendications précédentes, dans lequel les cellules sont des cellules de E. coli ayant une activité accrue d'aspartate transaminase, et présentant une constante d'équilibre supérieure à 10.

11. Procédé selon la revendication 10, dans lequel les cellules sont des cellules de E coli ATCC 11303.

**12.** Composition comprenant des cellules immobilisées de E. coli, douées d'une activité d'aspartate transaminase, un donneur d'amine comprenant de l'acide aspartique ou une combinaison de l'acide aspartique et de l'acide glutamique, ainsi que de l'acide phénylpyruvique ou du phénylpyruvate.

**13.** Composition selon la revendication 12, dans laquelle les cellules sont immobilisées à l'aide de polyazétidine.

**14.** Composition convenant pour servir à convertir l'acide phénylpyruvique ou un phénylpyruvate en de la phénylalanine, la composition comprenant des cellules immobilisées de E. coli douées d'une activité d'aspartate transaminase, lesdites cellules ayant été soumises à rupture ou à perméabilisation par un traitement chimique.

**Patentansprüche**

**1.** Verfahren zur Herstellung von Phenylalanin, welches das Kontaktieren von Phenylbrenztraubensäure oder Phenylpyruvat mit immobilisierten E. coli-Zellen mit Aspartat-Transaminase-Aktivität in Gegenwart eines Asparaginsäure oder eine Kombination von Asparaginsäure und Glutaminsäure umfassenden Amindonors umfaßt.

**2.** Verfahren nach Anspruch 1, bei dem die Zellen mit Hilfe eines Polyazetidinpolymers immobilisiert werden.

**3.** Verfahren nach Anspruch 2, bei dem die immobilisierten Zellen auf Kügelchen oder andere partikelförmige Materialien aufgetragen werden.

**4.** Verfahren nach einem der vorhergehenden Ansprüche, bei dem der Amindonor im Überschuß eingesetzt wird und der pH-Wert über 4 beträgt.

**5.** Verfahren nach Anspruch 4, bei dem der Überschuß an Amindonor mindestens 50% und der pH-Wert 5 bis 10 betragen.

**6.** Verfahren zur Herstellung von Phenylalanin, welches das Kontaktieren von Phenylbrenztraubensäure oder Phenylpyruvat mit immobilisierten E. coli-Zellen mit Aspartat-Transaminase-Aktivität in Gegenwart eines Asparaginsäure oder eine Kombination von Asparaginsäure und Glutaminsäure umfassenden Amindonors umfaßt, wobei die Zellen aufgeschlossen oder permeabilisiert worden sind, um den Kontakt zwischen dem Enzym und der Phenylbrenztraubensäure oder dem Phenylpyruvat zu fördern.

**7.** Verfahren nach Anspruch 6, bei dem die Zellen mittels Ultraschall aufgeschlossen worden sind.

**8.** Verfahren nach Anspruch 6, bei dem die Zellen durch Behandlung mit einem Tensid permeabilisiert werden.

**9.** Verfahren nach einem der vorhergehenden Ansprüche einschließlich der Zugabe von Pyridoxal-5-phosphat.

**10.** Verfahren nach einem der vorhergehenden Ansprüche, bei dem die Zellen E. coli-Zellen mit erhöhter Aspartat-Transaminase-Aktivität sind und eine Gleichgewichtskonstante von >10 aufweisen.

**11.** Verfahren nach Anspruch 10, bei dem die Zellen E. coli-Zellen des Stammes ATCC 11303 sind.

**12.** Zusammensetzung, welche immobilisierte E. coli-Zellen mit Aspartat-Transaminase-Aktivität, einen Asparaginsäure oder eine Kombination von Asparaginsäure und Glutaminsäuren umfassenden Amindonor, und Phenylbrenztraubensäure oder Phenylpyruvat umfaßt.

**13.** Zusammensetzung nach Anspruch 12, bei der die Zellen mit Polyazetidin immobilisiert sind.

**14.** Zusammensetzung, die zur Verwendung bei der Umwandlung von Phenylbrenztraubensäure oder Phenylpyruvat in Phenylalanin geeignet ist und immobilisierte E. coli-Zellen mit Aspartat-Transaminase-Aktivität umfaßt, wobei die Zellen durch chemische Behandlung aufgeschlossen oder permeabilisiert sind.